# EUROPÄISCHE PATENTSCHRIFT

(11) **EP 0 647 132 B1**
(45) Veröffentlichungstag und Bekanntmachung des Hinweises auf die Patenterteilung: **06.12.1995**
(21) Anmeldenummer: 93912638.9
(22) Anmeldetag: 24.06.1993
(51) Int. Cl.: A61K 9/127

(54) **GALENISCHE ZUSAMMENSETZUNG FÜR DIE TOPISCHE ANWENDUNG**
GALENIC COMPOSITION FOR TOPICAL USE
COMPOSITION GALENIQUE POUR APPLICATION TOPIQUE

(30) Priorität: 26.06.1992 DE 4221256
(43) Veröffentlichungstag der Anmeldung: 12.04.1995
(73) Patentinhaber: LANCASTER GROUP AG, 67059 Ludwigshafen (DE)
(72) Erfinder: GROSS, Udo, D-13059 Berlin (DE); ZASTROW, Leonhard, D-65205 Wiesbaden-Nordenstadt (DE); RÖDING, Joachim, D-65207 Wiesbaden-Rambach (DE); STANZL, Klaus, D-56323 Waldesch (DE)
(74) Vertreter: Walter, Wolf-Jürgen
(86) Internationale Anmeldenummer: DE9300574
(87) Internationale Veröffentlichungsnummer: WO9400110

(56) Entgegenhaltungen:
- WO-A-89/00848
- WO-A-89/08459
- WO-A-91/00110
- DE-A- 4 127 442

## Beschreibung

Die Erfindung betrifft galenische Zusammensetzungen auf Basis von Wirkstoffen, die mit Hilfe neuartiger Mikroaggregate als Träger in die Haut eingebracht werden. Die Anwendung relevanter Wirkstoffe ist auch am Auge möglich.

Es ist bekannt, pharmakologische Wirkstoffe liposomal zu verkapseln und als Dermatika topisch zu applizieren. In der DE-A-3542773 (J. Mueller) wird Triamcinolonacetonid als dermatologischer Wirkstoff vorgeschlagen. Normalerweise werden hydrophile Wirkstoffe aus einer wäßrigen Lösung liposomal eingeschlossen. Der Einschluß von wasserunlöslichen lipophilen Wirkstoffen ist dagegen auf diese Weise nicht möglich.

Es wurde deshalb u.a. vorgeschlagen, biologisch inerte Träger (carrier) z. B. Fluorcarbone zum Transport pharmakologischer Wirkstoffe zu verwenden. In EP-A-91313 (York) werden Fluorcarbone mit einem Dampfdruck zwischen 1 bis 16 mmHg vorgeschlagen, die als Träger von wasserempfindlichen bzw. wasserunlöslichen Wirkstoffen fungieren und auf der Haut oder am Auge angewendet werden. Nach dem Verdampfen des Fluorcarbons bleibt der Wirkstoff auf der Haut in einer dosierten Menge zurück. In EP-A-105584 (Yuha) wird ein Verfahren beschrieben, das mit Hilfe einer Fluorcarbon-Emulsion die Wirkung von Pharmaka für die Chemo- und Strahlentherapie von hypoxischen Krebszellen sensibilisiert. Die Wirkstoffe können sowohl hydrophiler als auch lipophiler Natur sein und gemeinsam bzw. unabhängig von der Fluorcarbon-Emulsion appliziert werden. Die Emulsion ist als eine konventionelle O/W-Emulsion anzusehen, die zur Emulgierung bekannte Emulgatoren verwendet.

Aus WO-A-89 00 848 ist ein Verfahren zur Behandlung und zur Verabreichung von Arzneimitteln auf die Haut bekannt, bei dem mit einer therapeutischen Menge Sauerstoff beladene Fluorcarbone sowie gegebenenfalls Antibiotika oder andere Arzneimittel im Gemisch auf die Haut aufgetragen werden. Die WO-A-89 08 459 beschreibt eine Perfluorcarbonemulsion mit Phospholipidvesikeln als Blutersatzstoff, bei der die Phospholipidmonomeren polymerisiert werden. In der WO-A-91 00110 werden Fluorcarbonemulsionen mit Phospholipiden offenbart, bei denen das Phospholipid gesättigte Kohlenstoffbindungen hat.

Der vorliegenden Erfindung liegt die Aufgabe zugrunde, pharmakologische Wirkstoffe in einem biologisch und chemisch inerten Träger für die therapeutische und diagnostische Anwendung auf der Haut oder am Auge oder zur systemischen Anwendung verfügbar zu machen und dabei ein tieferes Eindringen in die Haut oder den transkutanen Transport zu gewährleisten.

Erfindungsgemäß besteht eine galenische Zusammensetzung für die topische Anwendung aus einem Fluorcarbone enthaltenden lamellaren Phospholipid-Aggregat als Träger für pharmakologische Wirkstoffe, wobei der Anteil an Fluorcarbon oder Fluorcarbongemisch im Bereich von 1 bis 100 % Gewicht/Volumen liegt und das Phospholipid einen Phosphatidylcholingehalt von 30 bis 99 Gew.-% aufweist.

Überraschenderweise erfolgt eine Wechselwirkung zwischen dem Fluorcarbon, dem Wirkstoff und dem Phospholipid zu einem asymmetrischen lamellaren Aggregat mit besonderen Eigenschaften gegenüber dem dermalen System. Diese neuen Aggregate tragen im Unterschied zu den bekannten wäßrigen Liposomen (Vesikel) in ihrem Kern hydrophobe Fluorcarbone und bilden somit Monolayer mit inverser Anordnung, woran sich gegebenenfalls ein Aufbau von Bilayer-Schichten anschließt. Erfindungsgemäß weisen die lamellaren Aggregate daher eine asymmetrische Struktur auf im Unterschied zu den bekannten symmetrischen Vesikeln. Wegen dieser Besonderheit ihrer struktuellen Anordnung werden die neuartigen Aggregate als asymmetrische lamellare Träger bezeichnet. Ihre außergewöhnliche kolloidchemische Stabilität ist auf die lamellare Struktur und auf die Oberflächenladung der Aggregate zurückzuführen. Letztere ist auf die Auswahl geeigneter Phospholipide bzw. deren Mischungen natürlicher wie auch synthetischer Provenienz zurückzuführen. In erster Linie sind für eine vorteilhafte Wirkung in diesem Sinne elektrisch geladene Phospholipide wie Phosphatidylethanolamin (PE), N-Acyl-PE, N-Acetyl-PE oder Phosphatidsäure (PA) neben Phosphatidylcholin im Konzentrationsbereich von 30 bis 99 % verantwortlich.

In dem Kern dieser Aggregate befindet sich das Fluorcarbon als lipophiler Stoff. Die lamellare Struktur und ihre asymmetrische Anordnung wurde durch ³¹P-NMR und insbesondere durch spezielle elektronenmikroskopische Untersuchungen nachgewiesen. Die Teilchengrößen und -verteilungen wurden durch QLS-Untersuchungen bestimmt. Diese bewegen sich zwischen 50 und 1000 nm Teilchendurchmesser. Die Teilchengrößen sind abhängig von der Energieintensität des Homogenisierungsverfahrens.

Es können eine Vielzahl von Fluorcarbonen eingesetzt werden, z.B. aliphatische geradkettige und verzweigte Fluoralkane, mono- oder bicyclische und gegebenenfalls fluoralkylsubstituierte Fluorcycloalkane, perfluorierte aliphatische oder bicyclische Amine, Bis-(perfluoralkyl)-Ethene oder deren Gemische. Besonders bevorzugt sind solche Fluorcarbone wie Perfluordecalin, F-Butyltetrahydrofuran, Perfluortributylamin, Perfluoroctylbromid, Bis-Fluor(Butyl)ethen oder Bis-Fluor(Hexyl)ethen oder C₆-C₉-Perfluoralkane.

Dabei liegt der Anteil an Fluorcarbonen im Bereich von 1 bis 100 % w/v, vorzugsweise im Bereich von 40 bis 100 %. Ein besonders bevorzugter Bereich ist der von 70 bis 100 % w/v.

Als Phospholipide werden erfindungsgemäß natürliche Phospholipide wie Sojalecithin und Eilecithin, synthetische Phospholipide und/oder teilhydrierte Phospholipide eingesetzt, wobei bei diesen Phospholipiden der Gehalt an Phosphatidylcholin im Bereich von 30 bis 99 Gew.-%, insbesondere 70 bis 90 Gew.-% liegt, d.h. in den meisten Fällen erfolgt eine Anreicherung an Phophatidylcholin bei den Phospholipiden.

Neben Phosphatidylcholin können auch Lysolecithine im Konzentrationsbereich von 0,1 bis 5 Gew.-% vorhanden sein. Die angesprochene Wirkung der Phospholipide wird durch entsprechende negative Zeta-Potentiale und durch die Messung von Ladungsdichten (bei Titration mit einem kationischen Polyelektrolyten) verifiziert.

Die Abhängigkeit der Penetrationsgeschwindigkeit und der Eindringtiefe von der Teilchengröße der Aggregate konnte durch separate Untersuchungen im Tierexperiment mit markierten verkapselten Fluorcarbonen experimentell bestimmt werden. Danach wandern kleinere Teilchen schneller und tiefer in das Hautgewebe als größere Partikel. Die Auswahl von Fluorcarbonen bzw. deren Mischungen nach ihrer Lipidlöslichkeit (dargestellt durch ihre kritische Löslichkeitstemperatur CST in n-Hexan) erlaubt als ein weiteres wichtiges Kriterium die Regelung der Verweilzeit im Gewebe. Während z. B. Perfluortributylamin (F-TBA, CST 59° C) mit einem hohen CST-Wert und schlechter Lipidlöslichkeit eine größere Verweilzeit aufweist, wird im Gegensatz dazu Perfluordecalin (PFD, CST 22° C) aber auch F-Butyltetrahydrofuran, F-Hexan und andere entsprechend schneller aus dem Gewebe abgegeben. Mit Hilfe von Fluorcarbonmischungen lassen sich auf diese Weise gezielt Systeme mit gewünschten CST-Werten d. h. Lipid- und Membranlöslichkeiten bezüglich der beabsichtigten Anwendung herstellen.

Der Gehalt der Fluorcarbone als Wirkstoffträger in den lamellaren Aggregaten kann entsprechend dem Anwendungzweck zwischen 1 und 100 % w/v variieren. Als Fluorcarbone kommen insbesondere in Betracht:
aliphatische geradkettige und verzweigte Alkane mit 6 bis 12 Kohlenstoffatomen, wie z. B. Perfluorhexan, Perfluoroctan, Perfluornonan;
mono- oder bicyclische Cycloalkane, die gegebenenfalls F-alkylsubstituiert sind, wie z. B. Perfluormethylcyclohexan, Perfluordecalin;
aliphatische tertiäre Amine, N-haltige Polycyclen, wie z. B. Perfluortripropylamin, Perfluortributylamin, F-Cyclohexylmethylmorpholin;
Perfluorether, wie aliphatische Ether, F-Alkyl-Furane, bicyclische und substituierte bicyclische Ether mit 2 oder 3 Sauerstoffatomen im Molekül, wie z. B. Perfluordihexylether, Perfluorbutyltetrahydrofuran, Perfluorpolyether;
Perfluoralkylhalogenide, wie z. B. Perfluoroctylbromid, Perfluorhexylbromid, Perfluoroctylchlorid;
Bis-F(Alkyl)ethene, wie z. B. Bis-F(Butyl)ethen, Bis-F(Hexyl)ethen.

Unter dem hier verwendeten Begriff "Fluorcarbone" werden perfluorierte oder hochfluorierte Kohlenstoffverbindungen oder Gemische verstanden, die in der Lage sind, Gase wie O₂ und CO₂ zu transportieren. Hochfluorierte Kohlenwasserstoffverbindungen sind im Sinne dieser Erfindung solche, bei denen die meisten Wasserstoffatome durch Fluoratome ersetzt sind, wie z.B. die Bis-F(Alkyl)ethene, die nachweislich chemisch und biologisch inert und damit untoxisch sind. Dies wird meist dann erreicht, wenn etwa bis zu 90 % der Wasserstoffatome durch Fluoratome ersetzt sind. Bevorzugt im Sinne der vorliegenden Erfindung sind Fluorcarbone, bei denen wenigstens 95 % der Wasserstoffatome ersetzt sind, bevorzugter 98 % und am bevorzugtesten 100 %.

Es können auch einzelne Fluoratome durch andere Halogenatome wie Brom oder Chlor ersetzt sein.

Als Phospholipide kommen natürlich auftretende Phospholipide wie Soja- oder Eilecithin in Frage, sowie auch synthetisch herstellbare Lecithine (Phospholipide), die insgesamt als hautfreundlich und hautpflegend bekannt sind. Wegen der vorteilhaften Wirkung auf die Stabilität der asymmetrischen lamellaren Aggregate kommen Phospholipidmischungen mit einem Anteil von 30 bis 99 % an Phosphatidylcholin neben weiteren natürlich auftretenden Begleitprodukten zur Verwendung. Der Phospholipidgehalt in der kosmetischen Formulierung bewegt sich zwischen 0,5 und 20 Prozent.

Die neuen lamellaren Phospholipidaggregate haben die vorteilhafte Eigenschaft nach topischer Anwendung das Stratum corneum zu überwinden und in dem epidermalen und dermalen Bereich sowie im angrenzenden Gewebe den pharmazeutischen Wirkstoff verfügbar zu machen oder dem Gefäß zur systemischen Aufnahme bereitzustellen. Diese Penetrationseigenschaften werden erfindungsgemäß ausgenutzt, um Pharmaka in Wechselwirkung mit dem Fluorcarbon und dem Phospholipid in das Hautgewebe zu befördern und dort oder an anderer Stelle einen gewünschten therapeutischen oder diagnostischen Effekt zu erzielen. Dies erfolgt im Gegensatz zu den im Stand der Technik genannten Erfindungsbeschreibungen, die nicht den Transport der Fluorcarbone in tieferliegende Regionen der Haut gestatten. Die bekannten Verfahren sind in Bezug auf den beanspruchten Effekt wirkungslos.

Die erfindungsgemäße galenische Zusammensetzung enthält als pharmakologische Wirkstoffe insbesondere solche der folgenden Gruppe:
dermatologische Wirkstoffe, wie zum Beispiel Virustatika oder viruzide Arzneistoffe, Antimykotika, Heparine (z.B. HeparinCalcium, Heparin-Natrium, niedermolekulare Heparine), Antibiotika, Corticoide, Antiinfektiosia, Aknewirkstoffe, Lokalanästhetika, Antiphlogistika, Antihistaminika oder Antipsoriatika;
systemische Wirkstoffe, wie zum Beispiel nichtsteroidale Analgetika/Antirheumatika (z.B. Diclofenac-Natrium, Diclofenac-Diethylaminsalz, Etofenamat, Flufenaminsäure, 2-Hydroxyethylsalicylat, Ibuprofen, Indomethacin, Piroxicam), Opiatrezeptor-Agonisten und -Antagonisten (z.B. Buprenorphin, Fentanyl, Pentazocin, Pethidin, Tilidin, Tramadol, Naloxon), Histaminantagonisten (z.B. Bamipinlactat, Chlorphenoxamin-HCl, Clemastinhydrogenfumarat, Dimetindenmaleat, Pheniraminhydrogenmaleat), Insuline, regulatorische Peptide und ihre Hemmstoffe (z.B. Hypophysenvorderlappenhormone und ihre Hemmstoffe, Hypophyenhinterlappenhormone, Hypothalamushormone), Sedativa/Hypnotika (z.B. Diazepam);
Wirkstoffe der Gruppe Cytostakika, Cancerostatika, Immunmodulatoren, Vakzine.

Ein bevorzugter dermatologischer Wirkstoff ist beispielsweise Rosmarinsäure oder ein anderer in Pflanzen vorkommender viruzider oder virustatischer Wirkstoff. Ein bevorzugter systemischer Wirkstoff ist beispielsweise ein niedermolekulares oder hochmolekulares Heparin, ein Oligopeptid oder ein Polypeptid. Weitere bevorzugte Wirkstoffe sind Vitamine (E, A, B, C), Muramylpeptide, Doxorubicin, Gentamycin, Gramycidin, Dexamethason, Hydrocortison, Progesteron, Prednisolon bzw. davon abgeleitete Derivate und/oder Säure- bzw. Basenadditionssalze.

Mit relevanten Wirkstoffen und Wirkstoffkombinationen wird bei entsprechenden Indikationen eine antineoplastische Therapie, eine antimikrobielle und antivirale Therapie sowie weitere Therapieart möglich.

Im allgemeinen sind die Wirkstoffmengen in therapeutischer Hinsicht sehr gering, so daß z.B. für den Fall löslicher Wirkstoffe Löslichkeiten von 0,5 bis 12 g/100 ml Fluorcarbon ausreichend für eine medizinische Anwendung sind. Sollten diese Löslichkeiten nicht gegeben sein, so ist auch die Emulgierung über das noch nicht vollständig geklärte Zusammenwirken von Fluorcarbon und Phospholipid unter Anwendung bekannter Verfahren möglich, um zu der entsprechenden galenischen Zusammensetzung zu gelangen. Daher sind die Wirkstoffe in der aus bisheriger medizinischer Sicht ausreichenden Menge in den neuen Träger einarbeitbar.

Die Erfindung betrifft auch ein Verfahren zur Herstellung einer Phospholipide enthaltenden galenischen Zusammensetzung, das darin besteht, daß Phospholipide mit einem Gehalt an Phosphatidylcholin von 30 bis 99 Gew.-% emulgiert werden mit einem Fluorcarbon oder Fluorcarbongemisch, wobei ein pharmakologischer Wirkstoff oder eine Wirkstoffkombination in die Emulsion einbezogen wird, und wobei der Anteil an Fluorcarbon im Bereich von 1 bis 100 Prozent Gewicht/Volumen liegt, und die dabei erhaltenen asymmetrischen lamellaren Aggregate als Wirkstoffträger mit einer Teilchengröße von 50 bis 1000 nm in einen für die topische Anwendung geeigneten Exzipienten eingearbeitet werden.

Besonders bevorzugt ist dabei ein wasserlöslicher lipophiler Wirkstoff oder eine solche Wirkstoffkombination, die sich in dem Fluorcarbon lösen oder dispergiert/suspendiert darin vorliegen. Fluorcarbone sind grundsätzlich sehr hydrophobe organische Flüssigkeiten. Das breite Spektrum der chemischen Molekülstrukturen erlaubt jedoch eine Abstufung der lipophilen Eigenschaften, d.h. die unterschiedlichen Löslichkeitseigenschaften der Wirkstoffe können dann im Einzelfall einem ausgewählten Fluorcarbon angepaßt werden.

Berücksichtigt werden muß bei dem vorliegenden Herstellungsverfahren die oft komplizierte und empfindliche Molekülstruktur der Wirkstoffe mit unterschiedlichen Bindungsstabilitäten. Dies muß sowohl bei der Beladung der Fluorcarbone bzw. Fluorcarbongemische, bei der Wechselwirkung mit den Phospholipiden als auch beim Homogenisierungsprozeß besonders beachtet werden, da durch die Homogenisierung ein externer Energieeintrag erfolgt, mit dem die lamellaren Phospholipidaggregate erzeugt werden. Dieser Energieeintrag muß so bemessen sein, daß die Molekülstruktur der Pharmaka erhalten bleibt. Die Homogenisierung kann durch mechanische Mischer, Ultraschallmischer, Druckhomogenisatoren usw. erfolgen und ist vom Fachmann dem jeweiligen Pharmazeutikum anzupassen. Da Fluorcarbone zugleich als Sauerstoffträger dienen, kann zur Vermeidung von Autoxidationsprozessen der Einsatz von Antioxidantien angezeigt sein.

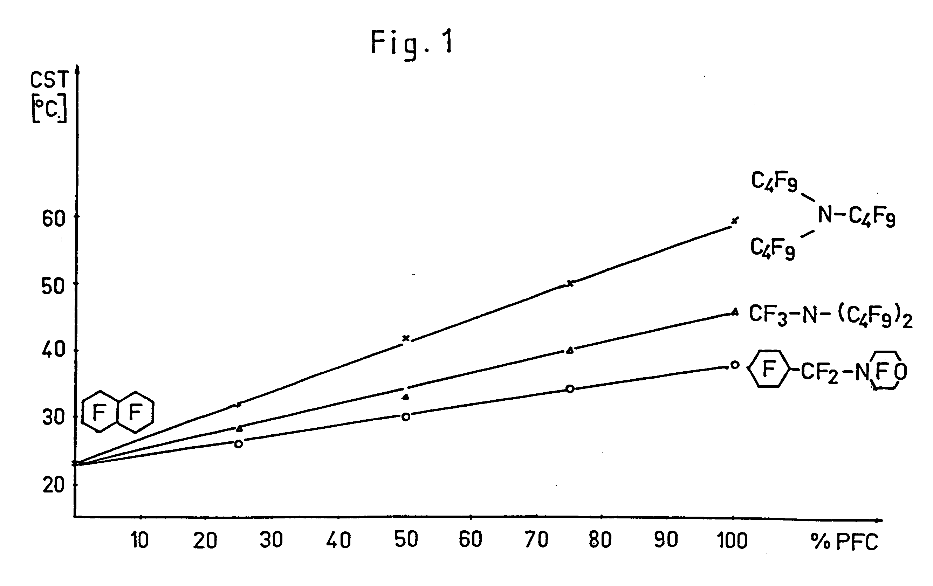

Die Erfindung soll nachstehend durch Beispiele näher erläutert werden. In der dazugehörigen Zeichnung bedeuten
Fig.1 Diagramm der kritischen Löslichkeitstemperaturen (CST) von Perfluorcarbongemischen in n-Hexan mit Perfluordecalin als Ausgangspunkt

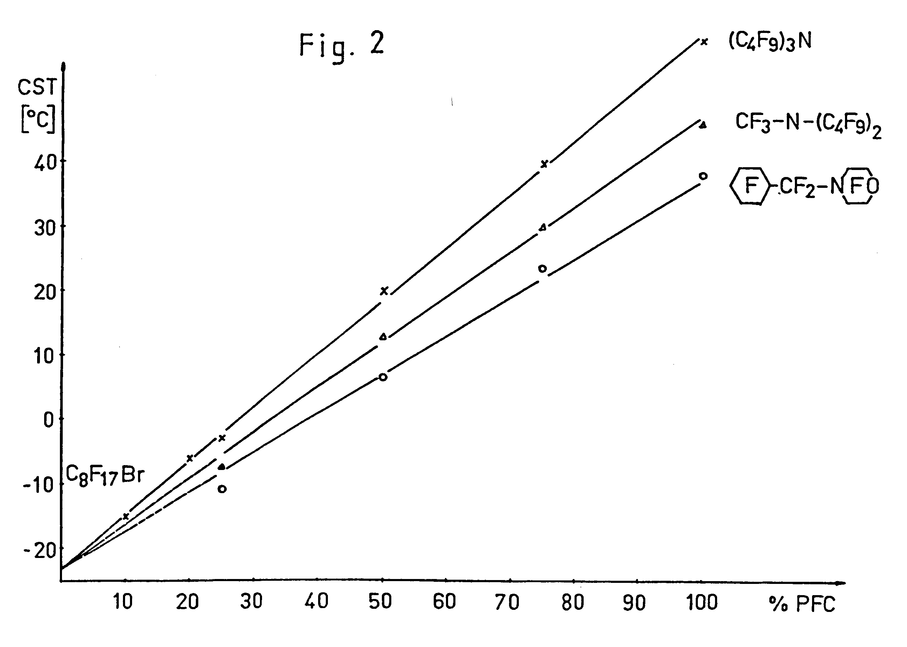

Fig.2 Diagramm der kritischen Löslichkeitstemperaturen von Perfluorcarbongemischen in n-Hexan mit F-Octylbromid als Ausgangspunkt.

In Tabelle 1 sind einige ausgewählte Fluorcarbone und ihre O₂-Löslichkeit, ihr Dampfdruck und die kritische Löslichkeitstemperatur dargestellt. Ausgehend von diesen Werten können für Gemische von Fluorcarbonen die gewünschten Charakteristika bei der Penetrierung in die Haut mit Hilfe der erfindungsgemäßen Zusammensetzung ausgewählt werden.

**Tabelle 1**

| Fluorcarbon | O₂-Löslichkeit [ml] O₂/100 ml FC] | Dampfdruck P_{37 °C} [mm Hg] | CST [°C] |
|---|---|---|---|
| Perfluoroctylbromid | 50 | 14 | -24,5 |
| Perfluordecalin | 40 | 12,5 | 22 |
| Bis-F(Butyl)ethen | 50 | 12,6 | 22,5 |
| F-cyclohexylmethylmorpholin | 42 | 4 | 38,5 |
| F-Tripropylamin | 45 | 18,5 | 43 |
| F-Dihexylether | 45 | 2 | 59 |
| F-Tributylamin | 40 | 1 | 59 |
| Perfluordecalin-F-Tributyl-amin 1/1 | 40 | 7 | 42 |
| Perfluorbutyltetrahydrofuran | 52 | 51 | 29 |
| F-methylcyclohexan | 57 | 180 | 8,2 |
| F-Hexan | 58 | 414 | 20 |

### Beispiel 1

Eine 10 %ige wäßrige Phospholipidlösung aus Sojalecithin und mit 40 % Phosphatidylcholin wurde zusammen mit einem Fluorcarbongemisch aus Perfluordecalin (90 %) und F-Dibutylmethylamin (10 %) und einem pharmakologischen Wirkstoff in einem Ultraschalldesintegrator unter Kühlung vermischt. Die dabei erhaltenen asymmetrischen lamellaren Phospholipidaggregate wiesen eine mittlere Teilchengröße von etwa 240 nm auf und enthielten den pharmakologischen Wirkstoff in Wechselwirkung mit dem Fluorcarbongemisch.

### Beispiel 2 Gel

Das in Beispiel 1 erhaltene Produkt wurde mit den einzelnen Bestandteilen vermischt, wobei die bei galenischen Verfahren üblichen Arbeitsweisen eingesetzt wurden. Dabei ergaben sich für die fertige galenische Zubereitung folgende Anteile

| | |
|---|---|
| asymmetrische lamellare Phospholipidaggregate | 30 % |
| Diazepam | 2 % |
| Polyacrylsäure | 1 % |
| TEA | 1 % |
| Konservierungsmittel | 0,8 % |
| Aqua dest. | ad 100 % |

### Beispiel 3 Alkoholische Lotion

Es wurde wie in Beispiel 2 verfahren, wobei folgende Bestandteile gegeben waren

| | |
|---|---|
| asymmetrische lamellare Phospholipidaggregate | 20 % |
| Ethanol | 16 % |
| Heparin-Na | 150.000 iE |
| Aqua dest. | ad 100 % |

### Beispiel 4 Creme

Es wurde wie in Beispiel 2 verfahren, wobei folgende Bestandteile gegeben waren

| | |
|---|---|
| asymmetrische lamellare Phospholipidaggregate | 20 % |
| Clotrimazol | 1 % |
| Vaselinum album | 79 % |

### Beispiel 5 Lotion

Es wurde wie in Beispiel 2 verfahren, wobei folgende Bestandteile gegeben waren

| | |
|---|---|
| asymmetrische lamellare Phospholipidaggregate | 20 % |
| Estradiol | 0,5 % |
| Polyacrylsäure | 0,2 % |
| TEA | 0,2 % |
| HCOH (37 %) | 0,2 % |
| Aqua dest. | ad 100 % |

## Patentansprüche

1. Galenische Zusammensetzung für die topische Anwendung mit Phosphollpiden und Fluorcarbonen, gekennzeichnet durch asymmetrische lamellare Aggregate, bestehend aus Phospholipiden mit einem Phosphatidylcholingehalt von 30 bis 99 Gew.-%, pharmakologischen Wirkstoffen und Fluorcarbonen oder Fluorcarbongemischen, wobei der Anteil an Fluorcarbon im Bereich von 1 bis 100 % Gewicht/Volumen liegt, in einem für die topische Anwendung geeigneten pharmazeutischen Trägerstoff.

2. Zusammensetzung nach Anspruch 1, dadurch gekennzeichnet, daß die lamellaren Aggregate von deren Fluorcarbon-Kern ausgehend eine asymmetrische, vorzugsweise dreischichtige Struktur aufweisen.

3. Zusammensetzung nach Anspruch 1 oder 2, dadurch gekennzeichnet, daß die Fluorcarbone aus der Gruppe ausgewählt sind, die aus aliphatischen geradkettigen und verzweigten Fluoralkanen, mono- oder bicyclischen gegebenenfalls fluoralkylsubstituierten Fluorcycloalkanen, perfluorierten aliphatischen oder bicyclischen Aminen, Bis-(perfluoralkyl)-ethenen oder deren Gemischen besteht.

4. Zusammensetzung nach Anspruch 3, dadurch gekennzeichnet, daß die Fluorcarbone aus der Gruppe ausgewählt sind, die aus Perfluordecalin, F-Butyltetrahydrofuran, Perfluortributylamin, Perfluoroctylbromid, Bis-Fluor(butyl)ethen oder C₆-C₉-Perfluoralkanen besteht.

5. Zusammensetzung nach einem der Ansprüche 1 bis 4, dadurch gekennzeichnet, daß der Anteil an Fluorcarbonen im Bereich von 20 bis 100 % Gewicht/Volumen liegt, vorzugsweise im Bereich von 40 bis 100 %, insbesondere im Bereich von 70 bis 100 %.

6. Zusammensetzung nach einem der Ansprüche 1 bis 5, dadurch gekennzeichnet, daß die Phospholipide ausgewählt sind aus der Gruppe, bestehend aus natürlichen Phospholipiden wie Sojalecithin und Eilecithin sowie synthetischen Phospholipiden, und/oder teilhydrierten Phospholipiden in einer Konzentration zwischen 0,5 und 20 %.

7. Zusammensetzung nach einem der Ansprüche 1 bis 6, dadurch gekennzeichnet, daß Phosphatidylcholin in einem Anteil von 70 bis 90 % vorhanden ist.

8. Zusammensetzung nach einem der Ansprüche 1 bis 7, dadurch gekennzeichnet, daß in der verwendeten Lipidfraktion neben Phosphatidylcholin Lysolecithine im Konzentrationsbereich von 1 bis 5 Gew.-% vorhanden sind.

9. Zusammensetzung nach Anspruch 1, dadurch gekennzeichnet, daß sie als Wirkstoffe einen oder mehrere der folgenden Gruppen enthält: dermatologische Wirkstoffe, systemische Wirkstoffe, Zytostatika, Cancerostatika, Immunmodulatoren, Vakzine.

10. Zusammensetzung nach Anspruch 9, dadurch gekennzeichnet, daß der Wirkstoff ein Arzneistoff aus der Gruppe Virustatika oder viruzide Arzneistoffe, Antimykotika, Heparine, Antibiotika, Corticoide, Antiinfektiosa, Aknewirkstoffe, Lokalanästhetika, Antiphlogistika, Antihistaminika oder Antipsoriatika ist

11. Zusammensetzung nach Anspruch 9, dadurch gekennzeichnet, daß der systemische Wirkstoff ein Arzneistoff aus der Gruppe der nichtsteroiden Analgetika/Antirheumatika, Opiatrezeptoragonisten, Opiatrezeptor-antagonisten, Heparine, Histaminantagonisten, Insuline, regulatorischen Peptide, Sedativa oder Hypnotika ist.

12. Zusammensetzung nach Anspruch 9, dadurch gekennzeichnet, daß der dermatologische Wirkstoff Rosmarinsäure oder ein anderer in Pflanzen vorkommender viruzider oder virustatischer Wirkstoff ist.

13. Zusammensetzung nach Anspruch 11, dadurch gekennzeichnet, daß der systemische Wirkstoff ein niedermolekulares oder hochmolekulares Heparin, ein Oligopeptid oder Polypeptid ist.

14. Verfahren zur Herstellung einer Phospholipide enthaltenden galenischen Zusammensetzung, dadurch gekennzeichnet, daß Phospholipide mit einem Phosphatidylcholingehalt von 30 bis 99 Gew.-% emulgiert werden mit einem Fluorcarbon oder Fluorcarbongemisch, wobei ein pharmakologischer Wirkstoff oder eine Wirkstoffkombination in die Emulsion eingebracht wird, und wobei der Anteil an Fluorcarbon im Bereich von 1 bis 100 Prozent Gewicht/Volumen liegt, und die dabei erhaltenen asymmetrischen lamellaren Aggregate als Wirkstoffträger mit einer Teilchengröße von 50 bis 1000 nm in einen für die topische Anwendung geeigneten Exzipienten eingearbeitet werden.

15. Verfahren nach Anspruch 14, dadurch gekennzeichnet, daß der Anteil an Fluorcarbonen im Bereich von 20 bis 100 % Gewicht/Volumen liegt, vorzugsweise im Bereich von 40 bis 100 % und der Anteil an Phosphatidylcholin im Bereich von 70 bis 90 Gew.-%.

16. Verwendung einer galenischen Zusammensetzung zur Herstellung eines Systems für die topische Applikation, enthaltend Phospholipide mit einem Phosphatidylcholingehalt von 30 bis 99 Gew.-%, pharmakologische Wirkstoffe und Fluorcarbone in Form asymmetrischer lamellarer Aggregate, wobei der Fluorcarbonanteil im Bereich von 0,2 bis 100 % Gewicht/Volumen liegt und das System in einem für die topische Anwendung typischen Träger wie Salben, Cremes, Lotionen, Pasten, Gele, Puder vorliegt, gegebenenfalls auf einem Verband oder einem Pflaster oder als Spray.

## Claims

1. Pharmaceutical composition for topical administration with phospholipids and fluorocarbons, characterised by asymmetric lamellar aggregates, consisting of phospholipids having a phosphatidylcholine content of 30 to 99 % by weight, pharmacological active compounds and fluorocarbons or fluorocarbon mixtures, the amount of fluorocarbon being in the range from 1 to 100 % weight/volume, in a pharmaceutical excipient suitable for topical administration.

2. Composition according to Claim 1, characterised in that the lamellar aggregates have an asymmetric, preferably 3-layer, structure originating from their fluorocarbon core.

3. Composition according to Claim 1, characterised in that the fluorocarbons are selected from the group which consists of aliphatic straight-chain and branched fluoroalkanes, mono- or bicyclic optionally fluoroalkyl-substituted fluorocycloalkanes, perfluorinated aliphatic or bicyclic amines, bis(perfluoroalkyl)ethenes and mixtures thereof.

4. Composition according to Claim 3, characterised in that the fluorocarbons are selected from the group which consists of perfluorodecalin, F-butyltetrahydrofuran, perfluorotributylamine, perfluorooctyl bromide, bis-fluoro(butyl)ethene or C₆-C₉-perfluoroalkanes.

5. Composition according to Claims 1, characterised in that the amount of fluorocarbons is in the range from 20 to 100 % weight/volume, preferably in the range from 40 to 100 %, in particular in the range from 70 to 100 %.

6. Composition according to Claim 1, characterised in that the phospholipids are selected from the group consisting of natural phospholipids such as soya lecithin and egg lecithin as well as synthetic phospholipids, and/or partially hydrogenated phospholipids, in a concentration between 0.5 and 20 %.

7. Composition according to Claim 1, characterised in that phosphatidylcholine is present in an amount from 70 to 90 %.

8. Composition according to Claim 1, characterised in that in the lipid fraction used, in addition to phosphatidylcholine, lysolecithins are present in the concentration range from 1 to 5 % by weight.

9. Composition according to Claim 1, characterised in that as active compounds it contains one or more of the following groups, dermatological active compounds, systemic active compounds, cytostatics, cancerostatics, immunomodulators and vaccines.

10. Composition according to Claim 9, characterised in that the active compound is a pharmaceutical from the group consisting of virustatics or virucidal pharmaceuticals, antimycotics, heparins, antibiotics, corticoids, antiinfectious agents, acne active compounds, local anaesthetics, antiinflammatories, antihistamines or antipsoriatic agents.

11. Composition according to Claim 9, characterised in that the systemic active compound is a pharmaceutical from the group consisting of the non-steroidal analgesics/antirheumatics, opiate receptor agonists, opiate receptor antagonists, heparins, histamine antagonists, insulins, regulatory peptides, sedatives or hypnotics.

12. Composition according to Claim 9, characterised in that the dermatological active compound is rosmarinic acid or another virucidal or virustatic active compound found in plants.

13. Composition according to Claim 11, characterised in that the systemic active compound is a low molecular weight or high molecular weight heparin, or an oligopeptide or polypeptide.

14. Process for the preparation of a phospholipidcontaining pharmaceutical composition, characterised in that phospholipids having a phosphatidylcholine content of 30 to 99 % by weight are emulsified with a fluorocarbon or fluorocarbon mixture, a pharmacological active compound or an active compound combination being incorporated into the emulsion, and the amount of fluorocarbon being in the range from 1 to 100 per cent weight/volume, and the asymmetric lamellar aggregates obtained in this way being incorporated into an excipient suitable for topical administration as active compound carriers having a particle size from 50 to 1000 nm.

15. Process according to Claim 14, characterised in that the amount of fluorocarbons is in the range from 20 to 100 % by weight/volume, preferably in the range from 40 to 100 %, and the amount of phosphatidylcholine in the phospholipid is in the range from 70 to 90 %.

16. Use of a pharmaceutical composition for producing a system by topical application containing phospholipids having a phosphatidylcholine content of 30 to 99 % by weight, pharmacological active compounds and fluorocarbons in the form of asymmetric lamellar aggregates, the fluorocarbon content being in the range from 0.2 to 100 % weight/volume and the system being present in a carrier which is typical for topical administration, such as ointments, creams, lotions, pastes, gels and powders, if appropriate on a dressing or a plaster or as a spray.

## Revendications

1. Composition galénique, contenant des phospholipides et des fluorocarbones, pour application topique, composition caractérisée par la présence d'agrégats lamellaires asymétriques consistant en des phospholipides ayant une teneur en phosphatidyl choline de 30 à 99 % en poids, en des substances pharmacologiquement actives et en des fluorocarbones ou des mélanges de fluorocarbones, la proportion du fluorocarbone se situant entre 1 et 100 % en poids/volume, dans une matière de support ou excipient pharmaceutique convenant pour une administration locale ou administration topique.

2. Composition selon la revendication 1, caractérisée en ce que les agrégats lamellaires présentent une structure asymétrique, avantageusement en trois couches, provenant de leurs noyaux fluorocarbonés.

3. Composition selon la revendication 1 ou 2, caractérisée en ce que les fluorocarbones sont choisis dans l'ensemble consistant en des fluoroalcanes aliphatiques, linéaires et ramifiés, des fluoroalcanes monocycliques ou bicycliques comportant éventuellement un ou des substituants fluoroalkyles, des amines fluorées, aliphatiques ou bicycliques, des bis-(perfluoroalkyl)-éthènes ou leurs mélanges.

4. Composition selon la revendication 3, caractérisée en ce que les fluorocarbones sont choisis dans l'ensemble consistant en de la perfluorodécaline, du F-butyltétrahydrofuranne, de la perfluorotributyl amine, du bromure de perfluoro-octyle, du bis-fluoro(butyl)éthène ou des perfluoro-alcanes en C₆ à C₉.

5. Composition selon l'une des revendication 1 à 4, caractérisée en ce que la proportion des fluorocarbones se situe entre 20 et 100 % en poids/volume, avantageusement entre 40 et 100 %, notamment entre 70 et 100 %.

6. Composition selon l'une des revendications 1 à 5, caractérisée en ce que les phospholipides sont choisis dans l'ensemble consistant en des phospholipides naturels comme de la lécithine de soja et de la lécithine d'oeuf, ainsi que des phospholipides synthétiques et/ou des phospholipides partiellement hydrogénés, présents en une concentration comprise entre 0,5 et 20 %.

7. Composition selon l'une des revendications 1 à 6, caractérisée en ce que la phosphatidyl choline est présente en une proportion de 70 à 90 %.

8. Composition selon l'une des revendications 1 à 7, caractérisée en ce que, dans la fraction lipidique utilisée, il y a en plus de la phosphatidyl choline des lysolécithines présentes en un domaine de concentration de 1 à 5 % en poids.

9. Composition selon la revendication 1, caractérisée en ce qu'elle contient comme substances actives des substances choisies parmi un ou plusieurs des groupes suivants : des substances dermatologiquement actives, des. substances actives sur le plan systémique, des cytostatiques, des cancérostatiques, des vaccins.

10. Composition selon la revendication 9, caractérisée en ce que la substance active est un médicament choisi dans l'ensemble formé par des virostatiques ou des médicaments viricides, des antimycoses, des héparines, des antibiotiques, des cortécoïdes, des produits anti-infectueux, des substances pouvant agir sur l'acné, des anasthésiques locaux, des fébrifuges ("antiphlogistiques"), des antihistaminiques ou des anti-psoriasis.

11. Composition selon la revendication 9, caractérisée en ce que la substance à activité systémique est un médicament choisi dans l'ensemble formé par des analgésiques/antirhumathismaux non stéréoïdiens, des agonistes de récepteurs d'opaciés, des antagonistes de récepteurs de produits opaciés, des héparines, des antagonistes de l'histamine, des insulines, des peptides régulateurs, des sédatifs ou des hypnotiques.

12. Composition selon la revendication 9, caractérisée en ce que la substance dermatologiquement active est une substance active formée par ou comportant de l'acide rosmarique pour un autre viricide présent dans des plantes ou un autre virostatique.

13. Composition selon la revendication 11 caractérisée en ce que la substance à activité systémique est une héparine à bas poids moléculaire ou à poids moléculaire élevé, un oligopeptide ou un polypeptide.

14. Procédé pour préparer une composition galénique contenant des phospholipides, caractérisé en ce qu'on émulsionne des phospholipides ayant une teneur en phosphatidyl choline de 30 à 99 % en poids avec un fluorocarbone ou un mélange de fluorocarbones, en incorporant une substance pharmacologiquement active ou une combinaison ou association de substances pharmacologiquement actives dans l'émulsion, et la proportion du fluorocarbone se situant entre 1 et 100 % en poids/volume, et les agrégats asymétriques lamellaires ainsi obtenus étant incorporés à titre de support ou excipient de substances actives, en une grosseur de particules de 50 à 1000 nm dans un excipient convenant pour une applicaiton topique.

15. Procédé selon la revendication 14, caractérisé en ce que la proportion des fluorocarbones se situe entre 20 et 100 % en poids/volume, avantageusement entre 40 et 100 %, et en ce que la proportion de la phosphatidyl choline se situe entre 70 et 90 % en poids.

16. Utilisation d'une composition galénique pour préparer un système pour application topique, ce système comportant des phospholipides ayant une teneur en phosphatidyl choline de 30 à 99 % en poids, des substances pharmacologiquement actives et des fluorocarbones sous forme d'agrégats lamellaires asymétriques, la proportion du ou des fluorocarbones se situant entre 0,2 et 100 % en poids/volume et le système étant présent dans un support typique pour application topique comme des baumes ou pommades, des crèmes, des lotions, des pâtes, des gels, de la poudre, éventuellement sur une bande de pansement ou sur un emplâtre ou sous forme de produit à nébuliser ou à projeter par pulvérisation ("spray").
